(19) Europäisches Patentamt
European Patent Office
Office européen des brevets

(11) EP 0 992 485 B1

(12) **EUROPEAN PATENT SPECIFICATION**

(45) Date of publication and mention of the grant of the patent:
**30.07.2003 Bulletin 2003/31**

(51) Int Cl.[7]: **C07C 69/734**, C09K 19/58, C07D 317/32 // C07M7:00

(21) Application number: **99118675.0**

(22) Date of filing: **22.09.1999**

(54) **Chiral compounds**

Chirale Verbindungen

Composés chiraux

(84) Designated Contracting States:
**DE GB**

(30) Priority: **02.10.1998 EP 98118646**

(43) Date of publication of application:
**12.04.2000 Bulletin 2000/15**

(73) Proprietor: **MERCK PATENT GmbH**
**64293 Darmstadt (DE)**

(72) Inventor: **Farrand, Louis, Dr.**
**Davyhulme, Manchester M41 7NT (GB)**

(56) References cited:
**EP-A- 0 351 746** **EP-A- 0 441 213**

- **DUBAL H R ET AL: "THREE CLASSES OF NEW CHIRAL DOPANTS: SYNTHESIS AND PHYSICAL QUALIFICATION AS DOPANTS FOR PRACTICAL FLC-MIXTURES" JAPANESE JOURNAL OF APPLIED PHYSICS,JP,PUBLICATION OFFICE JAPANESE JOURNAL OF APPLIED PHYSICS. TOKYO, vol. 27, no. 12, PART 02, - December 1988 (1988-12) page L2241-2244 XP000080785 ISSN: 0021-4922**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The invention relates to chiral compounds of formula Ic

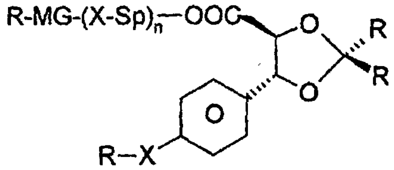

Ic

wherein R, MG, Sp, X and n are as defined in claim 1.

The invention also relates to a liquid crystalline mixture containing at least one chiral compound of formula Ic.

The invention also relates to a polymerizable liquid crystalline mixture comprising at least one chiral compound of formula Ic and at least one polymerizable mesogenic compound.

The invention furthermore relates to the use of chiral compounds of formula Ic and liquid crystalline mixtures and polymers obtained thereof in liquid crystal displays, such as STN, TN, AMD-TN, temperature compensation, guest-host, phase change or surface stabilized or polymer stabilized cholesteric texture (SSCT, PSCT) displays, in active and passive optical elements like polarizers, compensators, alignment layers, colour filters or holographic elements, in adhesives, synthetic resins with anisotropic mechanical properties, cosmetics, diagnostics, liquid crystal pigments, for decorative and security applications, in nonlinear optics, optical information storage or as chiral dopants,

The invention also relates to a liquid crystal display comprising a mixture comprising at least one chiral compound of formula Ic.

Chiral compounds can be used as dopants to induce or enhance a helical twist of the molecules of a liquid crystalline mixture that can be used for example in liquid crystal displays. The pitch p of the molecular helix in the first approximation, which is sufficient for most practical applications, is inversely proportional to the concentration c of the chiral dopant in the liquid crystal host mixture according to equation (1):

$$p=\frac{1}{HPC}\cdot\frac{1}{c} \qquad (1)$$

The proportionality factor is the helical twisting power (HTP) of the chiral dopant.

For many applications it is desirable to have LC mixtures that exhibit a twist. Among these are e.g. phase-change displays, guest-host displays, passive and active matrix TN and STN displays like AMD-TN, including such displays with temperature compensated characteristics, e.g. by appropriate selection of the cholesteric compounds according to the invention either alone or in combination with further chiral dopants. For these applications it is advantageous to have available a chiral dopant with a high HTP in order to reduce the amount of dopant needed to induce the desired pitch.

For some applications it is desired to have LC mixtures that exhibit a strong helical twist and thereby a short pitch length. For example in liquid crystalline mixtures that are used in selectively reflecting cholesteric displays, the pitch has to be selected such that the maximum of the wavelength reflected by the cholesteric helix is in the range of visible light. Another possible application are polymer films with a chiral liquid crystalline phase for optical elements, such as cholesteric broadband polarizers or chiral liquid crystalline retardation films.

As can be seen from equation (1), a short pitch can be achieved by using high amounts of dopant or by using a dopant with a high HTP.

However, the chiral dopants of prior art often exhibit low values of the HTP, so that high amounts of dopant are needed. This is a disadvantage because chiral dopants can be used only as pure enantiomers and are therefore expensive and difficult to synthesize.

Furthermore, when using chiral dopants of prior art in high amounts, they often negatively affect the properties of the liquid crystalline host mixture, such as e.g. the dielectric anisotropy $\Delta\varepsilon$, the viscosity, the driving voltage or the switching times.

Thus, there is a considerable demand for chiral compounds with a high HTP which are easy to synthesize, which can be used in low amounts, show improved temperature stability of the cholesteric pitch e.g. for utilizing a constant reflection wavelength and do not affect the properties of the liquid crystalline host mixture.

The invention has the aim of providing chiral compounds having these properties, but which do not have the disadvantages of the chiral dopants of the state of the art as discussed above.

Another aim of the invention is to extend the pool of chiral compounds that can be used as dopants available

to the expert.

It has been found that these aims can be achieved by providing chiral compounds according to formula Ic.

The inventive chiral compounds bear several advantages. Thus, they contain a chiral structure element exhibiting two centres of chirality and thus exhibit a high twisting power. Also, enantiomerically pure compounds of formula Ic are easy to prepare from cheap, readily available starting materials. The preparation methods are also suitable for large scale production. Furthermore, it is possible to prepare the R,S and S,R enantiomers which can be used to produce a cholesteric phase with either a right or a left handed helix. The availability of both helices can be a considerable advantage e.g. for the use in security film applications.

The EP 0 441 213-B1 describes chiral compounds comprising a 1,3-dioxolane group attached in 2-position to a mesogenic group, but does not specifically disclose compounds of formula Ic of the present invention.

EP 0 351 746-A1 describes chiral compounds comprising a 1,3-dioxolane group with a mesogenic group attached in 4-position, but does not disclose compounds of formula Ic of the present invention. Furthermore, the chiral compounds disclosed in EP 0 351 746-A1 do only exhibit a moderate HTP value.

One object of this invention are chiral compounds of formula Ic

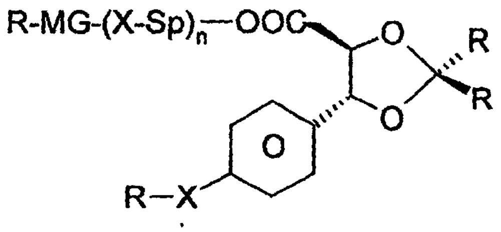

Ic

wherein

X is in each case independently -O-, -S-, -CO-, -COO-, -OCO-, -OCO-O-, -CO-NH-, -NH-CO-, $-OCH_2-$, $-CH_2O-$, -CH=CH-, -C≡C-, $-SCH_2-$, $-CH_2S-$, -CH=CH-COO-, -OOC-CH=CH- or a single bond,

Sp is in each case independently a spacer group with 1 to 20 C atoms,

n is 0 or 1,

MG is a mesogenic group, and

R is in each case independently H, CN, halogen or a straight-chain or branched alkyl radical with up to 25 C atoms which may be unsubstituted, mono- or polysubstituted by halogen or CN, it being also possible for one or more non-adjacent $CH_2$ groups to be replaced, in each case independently from one another, by -O-, -S-, -NH-, $-N(CH_3)-$, -CO-, -COO-, -OCO-, -OCO-O-, -S-CO-, -CO-S- or -C≡C- in such a manner that oxygen atoms are not linked directly to one another, or denoting $P-(Sp-X)_n-$, with Sp, X and n having one of the meanings given above and P being a polymerizable group.

Another object of the invention is a liquid crystalline mixture containing at least one chiral compound of formula Ic.

Another object of the invention is a polymerizable liquid crystalline mixture comprising at least one chiral compound of formula Ic and at least one polymerizable mesogenic compound having at least one polymerizable functional group. Another object of the invention is a chiral linear or crosslinked liquid crystalline polymer obtainable by polymerizing such a polymerizable mixture.

A further object of the invention is the use of a chiral compound, mixture or polymer as described above in liquid crystal displays, such as STN, TN, AMD-TN, temperature compensation, guest-host, phase change or surface stabilized or polymer stabilized cholesteric texture (SSCT, PSCT) displays, in active and passive optical elements like polarizers, compensators, alignment layers, colour filters or holographic elements, in adhesives, synthetic resins with anisotropic mechanical properties, cosmetics, diagnostics, liquid crystal pigments, for decorative and security applications, in nonlinear optics, optical information storage or as chiral dopants.

Yet another object of the invention is a liquid crystal display comprising a liquid crystalline mixture or a polymerizable liquid crystalline mixture comprising at least one chiral compound of formula Ic.

The inventive chiral compounds can additionally be mesogenic or even liquid crystalline, i.e. they can induce or enhance mesophase behaviour for example in admixture with other compounds, or even exhibit one or more mesophases themselves. It is also possible that the inventive compounds show mesophase behaviour only in mixtures with other compounds, or, in case of polymerizable compounds, when being (co)polymerized. Mesogenic inventive chiral compounds are especially preferred.

Further preferred are chiral compounds wherein MG is selected of formula II

$$-(A^1\text{-}Z)_m\text{-}A^2\text{-} \qquad \text{II}$$

wherein

Z is -O-, -S-, -CO-, -COO-, -OCO-, -CO-NH-, -NH-CO-, $-CH_2CH_2-$, $-OCH_2-$, $-CH_2O-$, $-SCH_2-$, $-CH_2S-$, -CH=CH-, -CH=CH-COO-, -OCO-CH=CH-, -C≡C- or a single bond,

$A^1$ and $A^2$ are in each case independently selected from

a) 1,4-phenylene in which, in addition, one or more CH groups may be replaced by N,

b) 1,4-cyclohexylene in which, in addition, one or two non-adjacent $CH_2$ groups may be replaced by O and/or S,

c) 1,3-dioxolane-4,5-diyl,

d) 1,4-cyclohexenylene, 1,4-bicyclo-(2,2,2)-octylene, piperidine-1,4-diyl, naphthalene-2,6-diyl, decahydronaphthalene-2,6-diyl, or 1,2,3,4-tetrahydronaphthalene-2,6-diyl,

it being possible for all these groups to be unsubstituted, mono- or polysubstituted with halogen, cyano or nitro groups or alkyl, alkoxy, alkylcarbonyl or alkoxycarbonyl groups with 1 to 7 C atoms, wherein one or more H atoms may be substituted by F or Cl, and

m is 0, 1, 2 or 3.

Further preferred are chiral compounds of formula Ic wherein

- R is alkyl or alkoxy with 1 to 12 C atoms,
- at least one of the groups R is denoting $P\text{-}(Sp\text{-}X)_n\text{-}$, with P, Sp, X and n having each independently one of the meanings given in formula Ic.

Of the inventive compounds especially preferred are those wherein MG incorporates one, two or three five- or six-membered rings.

Further preferred compounds are those in R is F, Cl, cyano, alkyl or alkoxy with 1 to 12 C atoms and MG is of formula II wherein Z is-COO-, -OCO-, $-CH_2\text{-}CH_2-$ or a single bond.

X is preferably -O-, -CO-, -COO-, -OCO-, $-CH_2O-$, $-OCH_2-$ or a single bond, in particular -O-, -COO-, -OCO- or a single bond.

Y is preferably -CO- or a single bond.

Particularly preferred compounds are those wherein MG is of formula II, and $A^1$ and $A^2$ are selected of 1,4-phenylene and trans-1,4-cyclohexylene, these rings being unsubstituted or substituted in 1 to 4 positions with F, Cl, CN or alkyl, alkoxy or alkoxycarbonyl with 1 to 4 C-atoms. From these preferred compounds, especially preferred are those wherein MG is a biphenyl or cyclohexylphenyl group.

Further preferred compounds are those wherein MG is comprising one or two 1,3-dioxolane-4,5-diyl rings, in particular wherein the 1,3-dioxolane-4,5-diyl groups are linked to an ester group.

A smaller group of preferred mesogenic groups MG of formula II is listed below. For reasons of simplicity, Phe in these groups is 1,4-phenylene, Phe L is a 1,4-phenylene group which is substituted by at least one group L, with L being F, Cl, CN or an optionally fluorinated alkyl, alkoxy or alkanoyl group with 1 to 4 C atoms, and Cyc is 1,4-cyclohexylene. The list of preferred mesogenic groups is comprising the formulae II-1 to II-27 as well as their mirror images

| | |
|---|---|
| - Phe- | II-1 |
| -Cyc- | II-2 |
| -PheL- | II-3 |
| -Phe-Z-Phe- | II-4 |
| -Phe-Z-Cyc- | II-5 |
| -Cyc-Z-Cyc- | II-6 |
| -PheL-Z-Phe- | II-7 |
| -PheL-Z-Cyc- | II-8 |
| -PheL-Z-PheL- | II-9 |
| -Phe-Z-Phe-Z-Phe- | II-10 |
| -Phe-Z-Phe-Z-Cyc- | II-11 |
| -Phe-Z-Cyc-Z-Phe- | II-12 |
| -Cyc-Z-Phe-Z-Cyc- | II-13 |
| -Phe-Z-Cyc-Z-Cyc- | II-14 |
| -Cyc-Z-Cyc-Z-Cyc- | II-15 |
| -Phe-Z-Phe-Z-PheL- | II-16 |
| -Phe-Z-PheL-Z-Phe- | II-17 |
| -PheL-Z-Phe-Z-PheL- | II-18 |
| -PheL-Z-PheL-Z-Phe- | II-19 |

-PheL-Z-PheL-Z-PheL- II-20

-Phe-Z-PheL-Z-Cyc- II-21

-Phe-Z-Cyc-Z-PheL- II-22

-Cyc-Z-Phe-Z-PheL- II-23

-PheL-Z-Cyc-Z-PheL- II-24

-PheL-Z-PheL-Z-Cyc- II-25

-PheL-Z-Cyc-Z-Cyc- II-26

-Cyc-Z-PheL-Z-Cyc- II-27

Bicyclic and tricyclic mesogenic groups MG are preferred.

Further preferred are compounds wherein MG is selected of formula II-7, II-8, II-9 or II-16 to II-27, and L is F, Cl, $CH_3$, $OCH_3$, $OCF_3$ or CN.

In the above list of preferred groups Z has the meaning given in formula I described above. Preferably Z is -COO-, -OCO-, $-CH_2CH_2-$-CH=CH-COO- or a single bond.

L is preferably F, Cl, CN, $NO_2$, $CH_3$, $C_2H_5$, $OCH_3$, $OC_2H_5$, $COCH_3$, $COC_2H_5$, $CF_3$, $OCF_3$, $OCHF_2$, $OC_2F_5$, in particular F, Cl, CN, $CH_3$, $C_2H_5$, $OCH_3$, $COCH_3$ and $OCF_3$, most preferably F, $CH_3$, $OCH_3$ and $COCH_3$.

Particularly preferred are chiral compounds wherein MG is selected from the following formulae and their mirror images

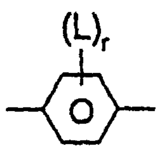

IIa

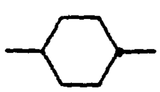

IIb

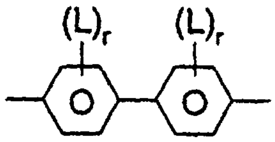

IIc

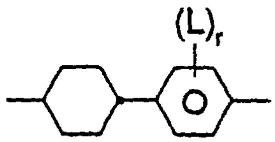

IId

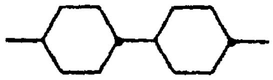

IIe

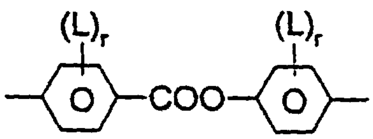

IIf

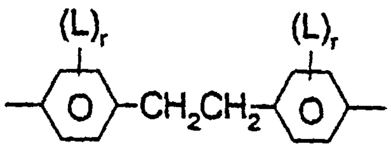

IIg

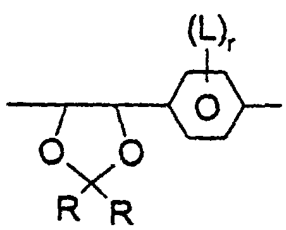

IIh

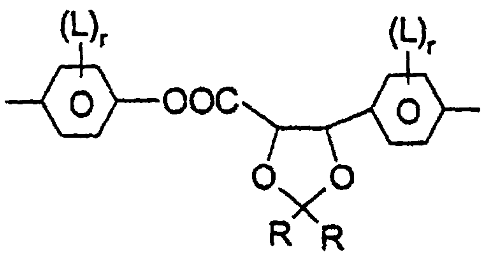

IIi

In these formulae R and L have the meaning given above and r is 0, 1 or 2

The group

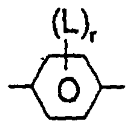

in these preferred formulae is very preferably denoting

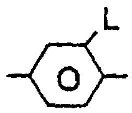 , 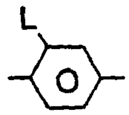 ,

or

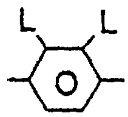 ,

furthermore

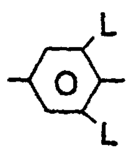 ,

with L having each independently one of the meanings given above.

R in the preferred compounds described above is particularly preferably CN, F, Cl, $OCF_3$ or an alkyl or alkoxy group with 1 to 12 C atoms. Straight-chain alkyl or alkoxy groups are especially preferred.

If R is an alkyl or alkoxy radical, i.e. where the terminal $CH_2$ group is replaced by -O-, this may be straight-chain or branched. It is preferably straight-chain, has 2, 3, 4, 5, 6, 7 or 8 carbon atoms and accordingly is preferably ethyl, propyl, butyl, pentyl, hexyl, heptyl, octyl, ethoxy, propoxy, butoxy, pentoxy, hexoxy, heptoxy, or octoxy, furthermore methyl, nonyl, decyl, undecyl, dodecyl, tridecyl, tetradecyl, pentadecyl, methoxy, nonoxy, decoxy, undecoxy, dodecoxy, tridecoxy or tetradecoxy, for example.

Oxaalkyl, i.e. where one $CH_2$ group is replaced by -O-, is preferably straight-chain 2-oxapropyl (=methoxymethyl), 2- (=ethoxymethyl) or 3-oxabutyl (=2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

In the chiral compounds of formula Ic R may be an achiral or a chiral group. In case of a chiral group it is preferably selected according to the following formula III:

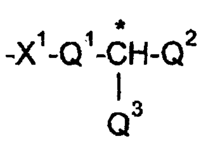

III

wherein

$X^1$ is -O-, -S-, -CO-, -COO-, -OCO-, -OCOO- or a single bond,

$Q^1$ is an alkylene or alkylene-oxy group with 1 to 10 C atoms or a single bond,

$Q^2$ is an alkyl or alkoxy group with 1 to 10 C atoms which may be unsubstituted, mono- or polysubstituted by halogen or CN, it being also possible for one or more non-adjacent $CH_2$ groups to be replaced, in each case independently from one another, by -C≡C-, -O-, -S-, -NH-, -N($CH_3$)-, -CO-, -COO-, -OCO-, -OCO-O-, -S-CO- or -CO-S- in such a manner that oxygen atoms are not linked directly to one another,

$Q^3$ is halogen, a cyano group or an alkyl or alkoxy group with 1 to 4 C atoms different from $Q^2$.

Preferred chiral groups R are 2-butyl (=1-methylpropyl), 2-methylbutyl, 2-methylpentyl, 3-methylpentyl, 2-ethylhexyl, 2-propytpentyl, 2-octyl, in particular 2-methylbutyl, 2-methylbutoxy, 2-methylpentoxy, 3-methylpentoxy, 2-ethylhexoxy, 1-methylhexoxy, 2-octyloxy, 2-oxa-3-methylbutyl, 3-oxa-43-oxa-4-methylpentyl, 4-methylhexyl, 2-nonyl, 2-decyl, 2-dodecyl, 6-methoxyoctoxy, 6-methyloctoxy, 6-methyloctanoyloxy, 5-methylheptyloxycarbonyl, 2-methylbutyryloxy, 3-methylvaleroyloxy, 4-methylhexanoyloxy, 2-chlorpropionyloxy, 2-chloro-3-methylbutyryloxy, 2-chloro-4-methylvaleryloxy, 2-chloro-3-methylvaleryloxy, 2-methyl-3-oxapentyl, 2-methyl-3-oxahexyl, 1-methoxypropyl-2-oxy, 1-ethoxypropyl-2-oxy, 1-propoxypropyl-2-oxy, 1-butoxypropyl-2-oxy, 2-fluorooctyloxy, 2-fluorodecyloxy for example.

In addition, chiral compounds of the formula Ic containing an achiral branched group R may occasionally be of importance, for example, due to a reduction in the tendency towards crystallization. Branched groups of this type generally do not contain more than one chain branch. Preferred achiral branched groups are isopropyl, isobutyl (=methylpropyl), isopentyl (=3-methylbutyl), isopropoxy, 2-methyl-propoxy and 3-methylbutoxy.

A particularly preferred embodiment of the present invention is related to polymerizable compounds of formula Ic, wherein at least one of the groups R is comprising a terminal polymerizable group P.

Of these compounds, especially preferred are those wherein one or two groups R are denoting P-(Sp-X)$_n$-. Very particularly preferred are compounds wherein the groups R denoting P-(Sp-X)$_n$- are adjacent to a mesogenic group MG.

Of the preferred compounds described above in particular preferred are those wherein n is 1.

Further preferred are compounds comprising at least one group R denoting P-(Sp-X)$_n$- wherein n is 0 and at least one group R denoting P-(Sp-X)$_n$- wherein n is 1.

Also preferred are compounds wherein in -(Sp-X)$_n$-MG-R n is 0.

The polymerizable group P is preferably selected from

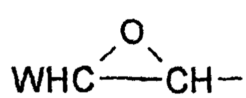,

$CH_2$=CW-COO-, WCH=CH-O-, and $CH_2$=CH-Phenyl-(O)$_k$-, with W being H, $CH_3$ or Cl and k being 0 or 1.

P is preferably a vinyl group, an acrylate group, a methacrylate group, a propenyl ether group or an epoxy group, especially preferably an acrylate or a methacrylate group.

In the event that one or more groups R are denoting P-(Sp-X)$_n$-, the two spacer groups Sp on both sides of the mesogenic group MG can be identical or different.

As for the spacer group Sp in formula Ic all groups can be used that are known for this purpose to the skilled in the art. The spacer group Sp is preferably a linear or branched alkylene group having 1 to 20 C atoms, in particular 1 to 12 C atoms, in which, in addition, one or more non-adjacent $CH_2$ groups may be replaced by -O-, -S-, -NH-, -N($CH_3$)-, -CO-, -O-CO-, -S-CO-, -O-COO-, -CO-S-, -CO-O-, -CH(halogen)-, -CH(CN)-, -CH=CH- or -C≡C-.

Typical spacer groups are for example -($CH_2$)$_o$-, -($CH_2CH_2O$)$_r$-$CH_2CH_2$-, -$CH_2CH_2$-S-$CH_2CH_2$- or -$CH_2CH_2$-NH-$CH_2CH_2$-,with o being an integer from 2 to 12 and r being an integer from 1 to 3.

Preferred spacer groups are ethylene, propylene, butylene, pentylene, hexylene, heptylene, octylene, nonylene, decylene, undecylene, dodecylene, octadecylene, ethyleneoxyethylene, methyleneoxybutylene, ethylenethioethylene, ethylene-N-methyl-iminoethylene, 1-methylalkylene, ethenylene, propenylene and butenylene for example.

Especially preferred are inventive chiral compounds of formula Ic wherein Sp is denoting an alkyl or alkoxy group with 2 to 6 C atoms. Straight-chain alkyl or alkoxy groups are especially preferred. In another preferred embodiment of the invention the chiral compounds of formula Ic comprise at least one spacer group Sp that is a chiral group of the formula IV:

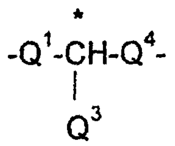

IV

wherein

$Q^1$ and $Q^3$ have the meanings given in formula III, and

$Q^4$ is an alkylene or alkylene-oxy group with 1 to 10 C atoms or a single bond, being different from $Q^1$.

The chiral structure element in formula Ic can be obtained by osmium-catalyzed asymmetric dihydroxylation of the corresponding olefin in the presence of $[DHQD]_2$PHAL (hydroquinidine 1,4-phthalazinediyl diether) or $[DHQ]_2$PHAL (hydroquinine 1,4-phthalazinediyl diether, both commercially available from Aldrich), in analogy to the methods described e.g. by K.B. Sharpless et al., J. Org. Chem. 57, 1992, 2768-2771 and S. Torii et al., J. Org. Chem. 61, 1996, 3055-3060. According to this method both the R,S and the S,R enantiomer can be synthesized.

Especially preferred are chiral compounds of formula Ic wherein the two chiral C atoms exhibit an S,R- or an R,S-configuration.

In particular, the inventive chiral compounds can be synthesized according to or in analogy to reaction scheme 1.

Scheme 1

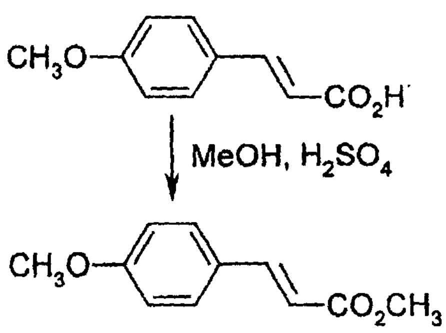


(1.1)

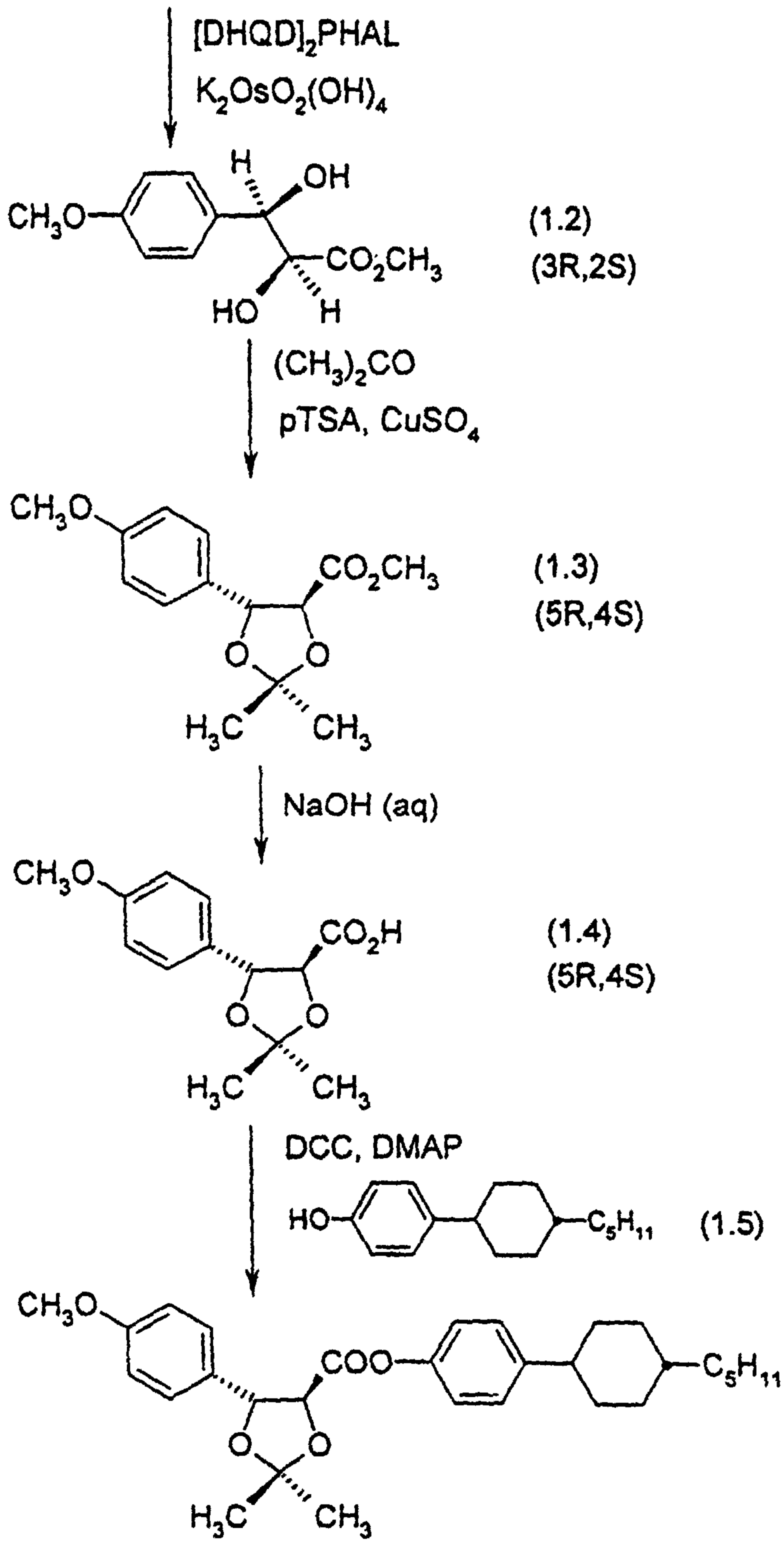


(1.2)
(3R,2S)

(1.3)
(5R,4S)

(1.4)
(5R,4S)

(1.5)

(1)
(5R,4S)

(1.4)

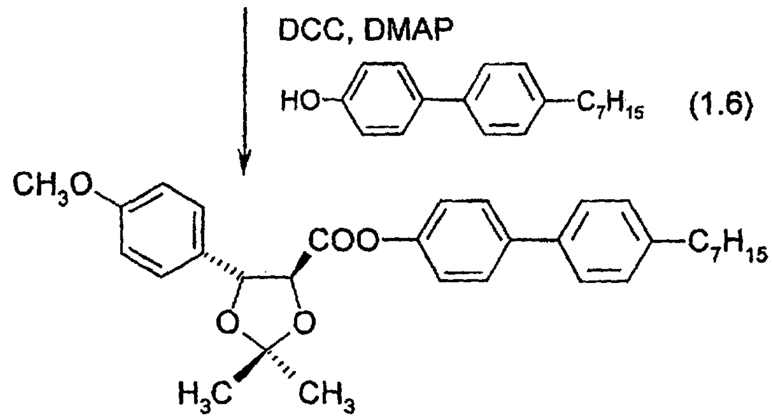


(1.6)

(2)
(5R,4S)

wherein DCC is denoting dicyclohexylcarbo-diimide, pTSA is p-toluene sulfonic acid, DMAP is N,N-dimethylamino-pyridine and DCM is dichloromethane.

Other compounds of formula Ic can be prepared in analogy to reaction scheme 1. For example, it is possible to react the intermediate (1.4) with a compound of the formula HO-($A^1$-Z)$_m$-$A^2$-R (1.7) according to reaction scheme 2.

## Scheme 2

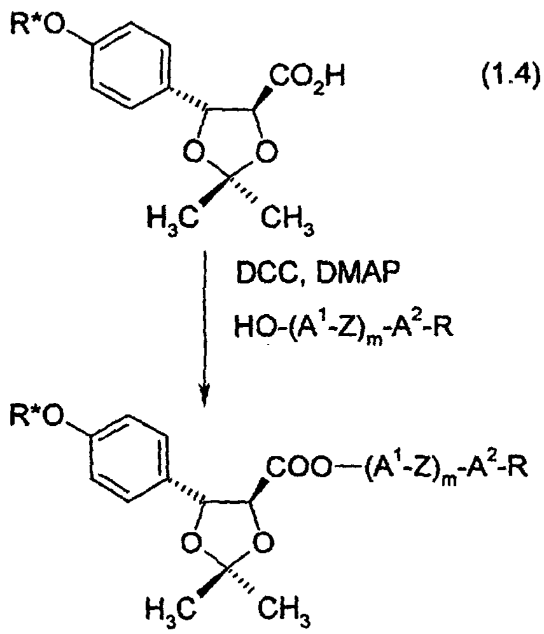


(1.4)

(1.7)

(1.8)

wherein R* has one of the meanings of R, R has the meaning of formula Ic and $A^1$, $A^2$, Z and m have the meanings of formula II.

It is further possible to prepare polymerizable compounds of formula Ic for example by reacting the intermediate (1.4) with a compound of formula HO-($A^1$-Z)$_m$-$A^2$-X-Sp-OOC-$CH_2CH_2Cl$ (1.9), and treating the resulting intermediate with $NEt_3$ according to reaction scheme 3.

## Scheme 3

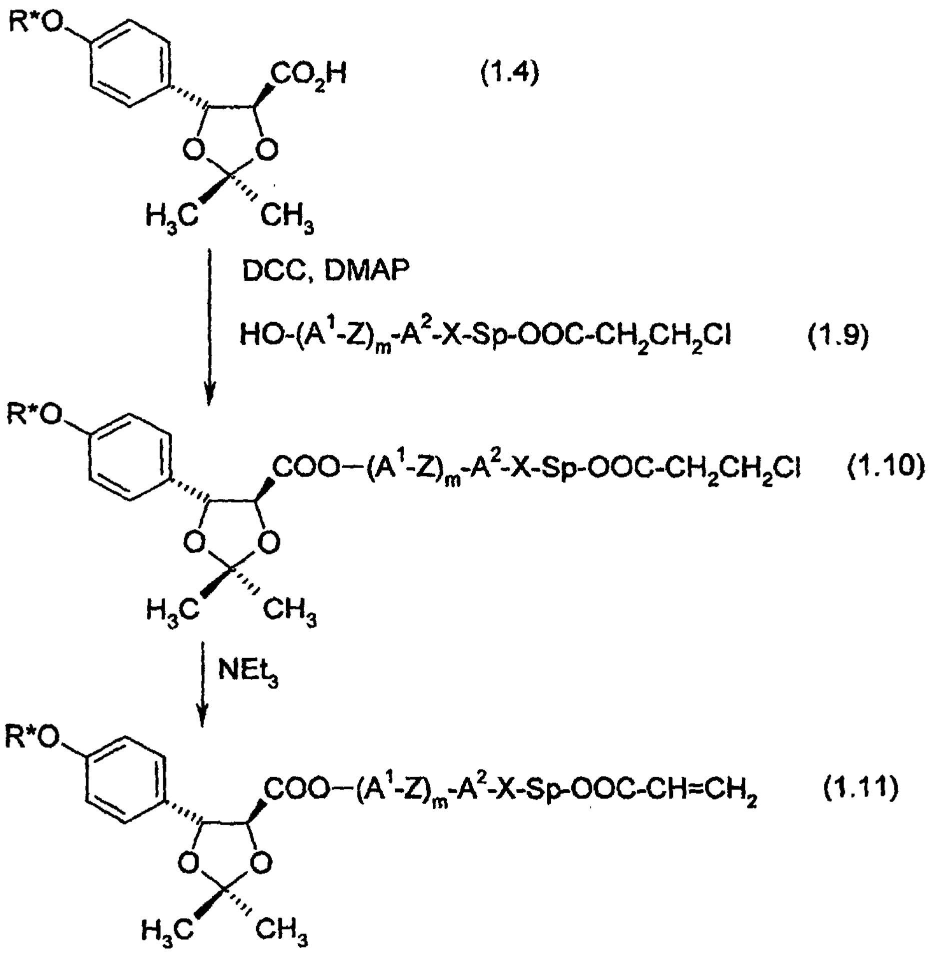


wherein R* has the meaning given above, X has the meaning of formula Ic, Sp has the meaning of formula Ic and $A^1$, $A^2$, Z and m have the meanings of formula II.

Other methods of preparation can be taken from the examples.

The inventive chiral compounds can be used in a liquid crystal mixture for displays exhibiting a twisted molecular structure of the liquid crystal matrix like, for example, supertwisted or active matrix liquid crystal displays, or in displays comprising a liquid crystal mixture with a chiral liquid crystalline phase, like for example chiral smectic or chiral nematic (cholesteric) mixtures for ferroelectric displays or cholesteric displays.

Thus, another object of the invention is a liquid crystalline mixture comprising at least one chiral compound of formula Ic.

Yet another object of the invention are cholesteric liquid crystal displays comprising cholesteric liquid crystalline media containing at least one chiral compound of formula Ic.

The inventive chiral compounds of formula Ic exhibit high values of the HTP. Thus liquid crystalline mixtures with a high helical twist, i.e. a short cholesteric pitch, can be prepared by using the inventive compounds, or otherwise a liquid crystalline mixture with a moderate helical twist can be achieved already when using the inventive compounds as dopants in low amounts.

The high HTP values of the inventive compounds makes them also suitable to be used in combination with other compounds for the temperature compensation of the properties of liquid crystal mixtures, such as the cholesteric pitch, and of the properties of displays, e.g. such as the threshold voltage.

In a preferred embodiment of the invention the chiral compounds show a strong temperature dependence of the HTP in nematic liquid crystal mixtures.

The inventive compounds are furthermore advantageous because they are affecting the physical properties

of the liquid crystalline mixture only to a minor extent.

Thus, when admixing the chiral compounds of formula Ic for example to a liquid crystalline mixture with positive dielectric anisotropy that is used in a liquid crystal display, $\Delta\varepsilon$ is being only slightly reduced and the viscosity of the liquid crystalline mixture is increased only to a small extent. This leads to lower voltages and improved switching times of the display when compared to a display comprising conventional dopants.

In a particularly preferred embodiment of the invention the chiral compounds show a small temperature dependence of the HTP in nematic liquid crystal mixtures.

The liquid crystalline mixture according to the invention comprises preferably 0.001 to 15 %, in particular 0.01 to 8 % and very particularly preferably 0.1 to 5 % by weight of chiral compounds of formula Ic.

The liquid crystalline mixture according to the invention preferably comprises 1 to 3 chiral compounds of formula Ic.

For temperature compensation applications as described above the liquid crystalline mixture preferably contains a chiral component which contains at least one chiral compound of formula Ic.

In a preferred embodiment of the invention the liquid crystalline mixture is consisting of 2 to 25, preferably 3 to 15 compounds, at least one of which is a chiral compound of formula Ic. The other compounds are preferably low molecular weight liquid crystalline compounds selected from nematic or nematogenic substances, for example from the known classes of the azoxybenzenes, benzylidene-anilines, biphenyls, terphenyls, phenyl or cyclohexyl benzoates, phenyl or cyclohexyl esters of cyclohehexanecarboxylic acid, phenyl or cyclohexyl esters of cyclohexylbenzoic acid, phenyl or cyclohexyl esters of cyclohexylcyclohexanecarboxylic acid, cyclohexylphenyl esters of benzoic acid, of cyclohexanecarboxylic acid and of cyclohexylcyclohexanecarboxylic acid, phenylcyclohexanes, cyclohexylbiphenyls, phenylcyclohexylcyclohexanes, cyclohexylcyclohexanes, cyclohexylcyclohexenes, cyclohexylcyclohexylcyclohexenes, 1,4-bis-cyclohexylbenzenes, 4,4'-bis-cyclohexylbiphenyls, phenyl- or cyclohexylpyrimidines, phenyl- or cyclohexylpyridines, phenyl- or cyclo-hexylpyridazines, phenyl- or cyclohexyldioxanes, phenyl- or cyclo-hexyl-1,3-dithianes, 1,2-diphenyl-ethanes, 1,2-dicyclohexylethanes, 1-phenyl-2-cyclohexylethanes, 1-cyclohexyl-2-(4-phenylcyclohexyl)-ethanes, 1-cyclohexyl-2-biphenyl-ethanes, 1 -phenyl2-cyclohexyl-phenylethanes, optionally halogenated stilbenes, benzyl phenyl ether, tolanes, substituted cinnamic acids and further classes of nematic or nematogenic substances. The 1,4-phenylene groups in these compounds may also be laterally mono- or difluorinated.

The liquid crystalline mixture of this preferred embodiment is based on the achiral compounds of this type.

The most important compounds that are posssible as components of these liquid crystalline mixtures can be characterized by the following formula

R'-L'-G'-E-R"

wherein L' and E, which may be identical or different, are in each case, independently from one another, a bivalent radical from the group formed by -Phe-, -Cyc-, -Phe-Phe-, -Phe-Cyc-, -Cyc-Cyc-, -Pyr-, -Dio-, -B-Phe- and -B-Cyc- and their mirror images, where Phe is unsubstituted or fluorine-substituted 1,4-phenylene, Cyc is trans-1,4-cyclohexylene or 1,4-cyclohexenylene, Pyr is pyrimidine-2,5-diyl or pyridine-2,5-diyl, Dio is 1,3-dioxane-2,5-diyl abd B is 2-(trans-1,4-cyclohexyl)ethyl, pyrimidine-2,5-diyl, pyridine-2,5-diyl or 1,3-dioxane-2,5-diyl.

G' in these compounds is selected from the following bivalent groups -CH=CH-, -N(O)N-, -CH=CY-, -CH=N(O)-, -C=C-, $-CH_2-CH_2-$, -CO-O-, $-CH_2-O-$, -CO-S-, $-CH_2-S-$, -CH=N-, -COO-Phe-COO- or a single bond, with Y being halogen, preferably chlorine, or -CN.

R' and R" are, in each case, independently of one another, alkyl, alkenyl, alkoxy, alkenyloxy, alkanoyloxy, alkoxycarbonyl or alkoxycarbonyloxy with 1 to 18, preferably 3 to 12 C atoms, or alternatively one of R' and R" is F, $CF_3$, $OCF_3$, Cl, NCS or CN.

In most of these compounds R' and R" are, in each case, independently of each another, alkyl, alkenyl or alkoxy with different chain length, wherein the sum of C atoms in nematic media generally is between 2 and 9, preferably between 2 and 7.

Many of these compounds or mixtures thereof are commercially available. All of these compounds are either known or can be prepared by methods which are known per se, as described in the literature (for example in the standard works such as Houben-Weyl, Methoden der Organischen Chemie [Methods of Organic Chemistry], Georg-Thieme-Verlag, Stuttgart), to be precise under reaction conditions which are known and suitable for said reactions. Use may also be made here of variants which are known per se, but are not mentioned here.

The inventive compounds are in particular useful for anisotropic polymer gels and for low molar mass or polymerizable or polymerized cholesteric liquid crystalline mixtures for cholesteric displays, such as for example phase change displays or surface stabilized or polymer stabilized cholesteric texture displays (SSCT, PSCT).

A further advantage of the chiral compounds according to the invention is that cholesteric liquid crystalline mixtures or materials comprising these compounds exhibit a low temperature dependence of the reflection wavelength

dλ/dT (T = temperature, λ = reflection wavelength maximum).

Cholesteric displays are described for example in WO 92/19695, WO 93/23496, US 5,453,863 or US 5,493,430, with the entire disclosure of these documents being introduced into this application by way of reference.

Furthermore, anisotropic polymer gels and displays comprising them are disclosed for example in DE 195 04 224 and GB 2 279 659.

It has been found that PSCT displays comprising the inventive compounds have reduced response times, lower voltages and improved contrast compared to displays comprising conventional dopants, like e.g. R 811 or CB 15, that are commercially available by Merck KGaA (Darmstadt, Germany). For example, PSCT displays in which the conventional dopants are replaced by chiral compounds of according to the present invention can show reduced switching time.

Cholesteric films made by using the inventive compounds instead of prior art dopants show improved brightness, leading to a better contrast between the coloured planar texture and the almost clear focal conic state which is made black using a black backplate.

The inventive chiral compounds and polymerizable liquid crystalline mixtures comprising these compounds are also particularly useful for the preparation of anisotropic polymer films with a chiral liquid crystalline phase, such as cholesteric or chiral smectic polymer films, in particular films that exhibit helically twisted molecular structure with uniform planar orientation, i.e. wherein the helical axis is oriented perpendicular to the plane of the film.

For example, oriented cholesteric polymer films can be used as broad waveband reflective polarizers, as described e.g. in EP 0 606 940, as colour filters, for security markings, or for the preparation of liquid crystal pigments. I. Heynderickx and D.J. Broer in Mol.Cryst.Liq.Cryst. 203, 113-126 (1991) describe crosslinked cholesteric polymer films that are made of liquid crystalline diacrylates and contain a low molecular weight chiral dopant.

It has been found that cholesteric polymer films made by using the inventive chiral compounds are brighter compared to films comprising dopants of prior art like e.g. R 811 or CB 15 as mentioned above.

For the preparation of anisotropic polymer gels or oriented polymer films, the liquid crystalline mixture should comprise at least one polymerizable compound, preferably a polymerizable mesogenic compound, in addition to chiral compounds of formula Ic.

Thus, another object of the invention are polymerizable liquid crystalline mixtures comprising at least one chiral compound of formula Ic and at least one polymerizable mesogenic compound.

Suitable polymerizable mesogenic compounds are described for example in WO 93/22397; EP 0 261 712; DE 19504224; DE 4408171 or DE 4405316. The compounds disclosed in these documents, however, are to be regarded merely as examples that shall not limit the scope of this invention.

Furthermore, typical examples representing polymerizable mesogenic compounds are shown in the following list of compounds, which should, however, be taken only as illustrative and is in no way intended to restrict, but instead to explain the present invention:

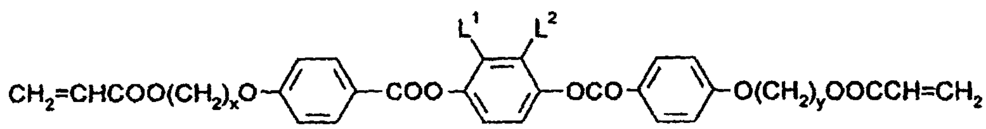

(V1)

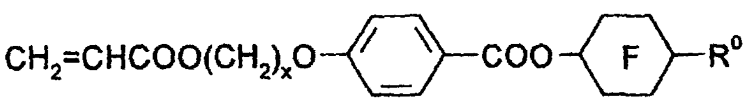

(V2)

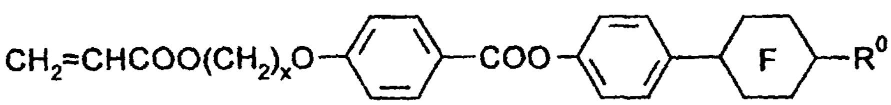

(V3)

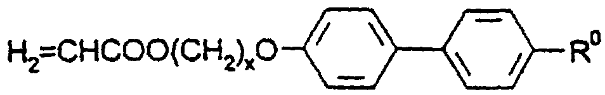

(V4)

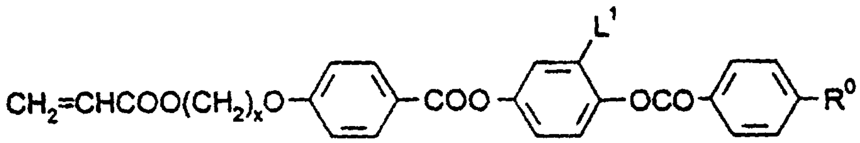

(V5)

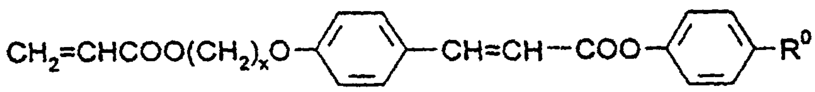

(V6)

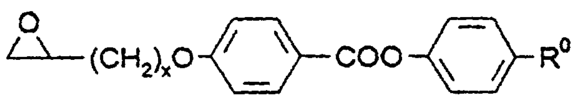

(V7)

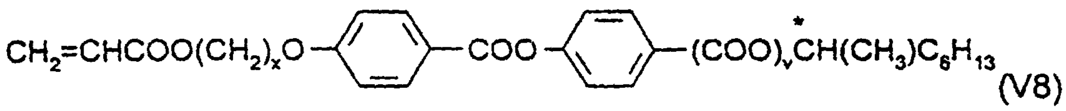

(V8)

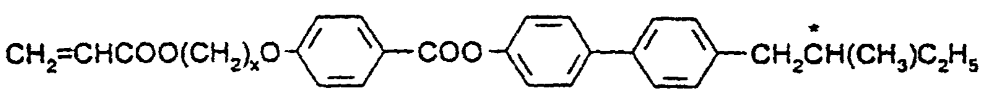

(V9)

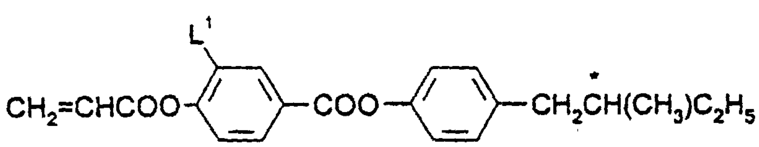

(V10)

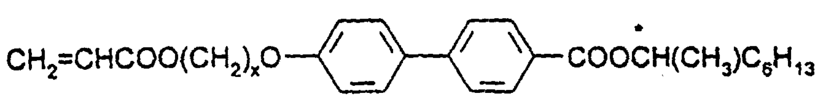

(V11)

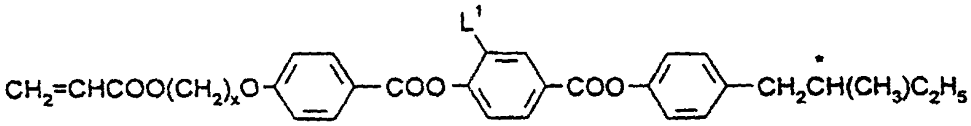

(V12)

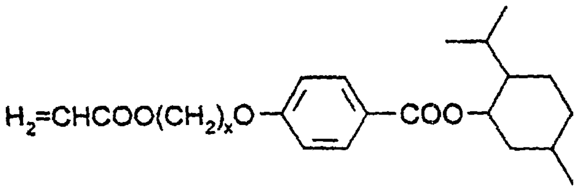

(V13)

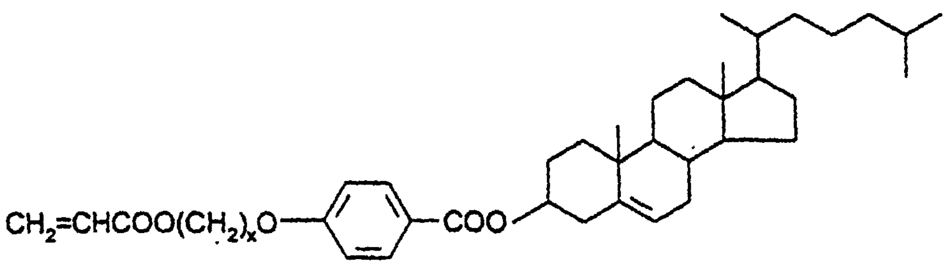

(V14)

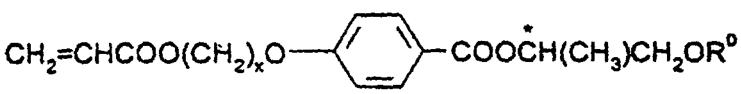

(V15)

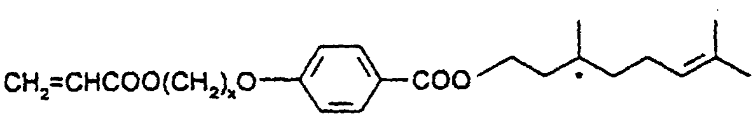

(V16)

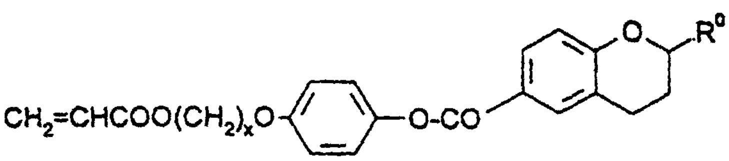

(V17)

In these compounds x and y are each independently 1 to 12 F is a 1,4-phenylene or 1,4-cyclohexylene group, $R^o$ is halogen, cyano or an optionally halogenated alkyl or alkoxy group with 1 to 12 C atoms and $L^1$ and $L^2$ are each independently H, F, Cl, CN, or an optionally halogenated alkyl, alkoxy or alkoxycarbonyl group with 1 to 7 C atoms.

The polymerizable mesogenic compounds of formula V1 - V17 can be prepared by methods which are known

per se and which are described in the documents cited above and, for example, in standard works of organic chemistry such as, for example, Houben-Weyl, Methoden der organischen Chemie, Thieme-Verlag, Stuttgart.

The polymerizable mesogenic compounds of formula V1 - V17 can be mono- or bifunctional, i.e. they can have one or two polymerizable functional groups. at least one monofunctional and at least one bifunctional polymerizable compound of formulae V1-V17.

In another preferred embodiment the polymerizable liquid crystalline mixtures comprise at least one inventive chiral compound and at least two monofunctional compounds of formulae V1-V17.

It is also possible that the polymerizable liquid crystalline mixture comprises one or more polymerizable compounds of formula Ic instead of or in addition to the polymerizable mesogenic compounds of formulae V1-V17.

Thus, another object of the invention are polymerizable liquid crystalline mixtures comprising at least one chiral compound of formula Ic comprising at least one polymerizable functional group.

In a preferred embodiment the polymerizable liquid crystalline mixtures comprise at least one chiral compound of formula Ic comprising one polymerizable functional group.

In another preferred embodiment the polymerizable liquid crystalline mixtures comprise at least one chiral compound of formula Ic comprising two polymerizable functional groups.

Another object of the invention is an anisotropic polymer film with an oriented chiral liquid crystalline phase obtainable by (co)polymerizing a liquid crystalline mixture comprising at least one chiral compound of formula Ic and at least one polymerizable mesogenic compound preferably selected of formula V1-V17 and/or at least one polymerizable chiral compound of formula Ic.

To prepare anisotropic polymer film with a chiral liquid crystalline phase with uniform orientation the inventive liquid crystalline mixtures, for example, are coated onto a substrate, aligned and polymerized in situ by exposing them to heat or actinic radiation.

Alignment and curing are preferably carried out in the liquid crystalline phase of the liquid crystalline mixtures.

Actinic radiation means irradiation with light, like UV light, IR light or visible light, irradiation with X-rays or gamma rays or irradiation with high energy particles, such as ions or electrons. As a source for actinic radiation for example a single UV lamp or a set of UV lamps can be used. Another possible source for actinic radiation is a laser, like e.g. a UV laser, an IR laser or a visible laser.

For example, when polymerizing by means of UV light, a photoinitiator can be used that decomposes under UV irradiation to produce free radicals or ions that start the polymerization reaction.

It is also possible to use a cationic photoinitiator, when curing reactive mesogens with for example vinyl and epoxide reactive groups, that photocures with cations instead of free radicals.

As a photoinitiator for radical polymerization for example the commercially available Irgacure 651, Irgacure 184, Darocure 1173 or Darocure 4205 (all from Ciba Geigy AG) can be used, whereas in case of cationic photopolymerization the commercially available UVI 6974 (Union Carbide) can be used.

Preferably the polymerizable liquid crystalline mixtures comprising polymerizable chiral compounds of formula Ic and/or polymerizable mesogenic compounds of formulae V1-V17 additionally comprise 0.01 to 10 %, in particular 0.05 to 8 %, very preferably 0.1 to 5 % by weight of a photoinitiator, especially preferably a UV-photoinitiator.

In a preferred embodiment of the invention the polymerization of the polymerizable mesogenic material is carried out under an atmosphere of inert gas, preferably under a nitrogen atmosphere.

As a substrate for example a glass or quarz sheet as well as a plastic film or sheet can be used. It is also possible to put a second substrate on top of the coated mixture prior to, during and/or after polymerization. The substrates can be removed after polymerization or not. When using two substrates in case of curing by actinic radiation, at least one substrate has to be transmissive for the actinic radiation used for the polymerization.

Isotropic or birefringent substrates can be used. In case the substrate is not removed from the polymerized film after polymerization, preferably isotropic substrates are used.

Preferably at least one substrate is a plastic substrate such as for example a film of polyester such as polyethyleneterephthalate (PET), of polyvinylalcohol (PVA), polycarbonate (PC) or triacetylcellulose (TAC), especially preferably a PET film or a TAC film. As a birefringent substrate for example an uniaxially stretched plastic film can be used. For example PET films are commercially available from ICI Corp. under the trade name Melinex.

In a preferred embodiment of the present invention, the inventive mixture of the polymerizable liquid crystalline mixture comprising a chiral compound of formula Ic is coated as a thin layer on a substrate or between substrate, and is preferably aligned in its chiral mesophase, eg. the cholesteric or chiral smectic phase, to give a planar orientation, i.e. an orientation so that the axis of the molecular helix extends transversely to the layer.

A planar orientation can be achieved for example by shearing the mixture, e.g. by means of a doctor blade. It is also possible to apply an alignment layer, for example a layer of rubbed polyimide or sputtered $SiO_x$, on top of at least one of the substrates.

In another preferred embodiment, a second substrate is put on top of the coated material. In this case, the shearing caused by putting together the two substrates is sufficient to give good alignment. It is also possible to apply

an electric or magnetic field to the coated mixture.

In some cases it is of advantage to apply a second substrate not only to aid alignment of the polymerizable mixture but also to exclude oxygen that may inhibit the polymerization. Alternatively the curing can be carried out under an atmosphere of inert gas. However, curing in air is also possible using suitable photoinitiators and high lamp power. When using a cationic photoinitiator oxygen exclusion most often is not needed, but water should be excluded.

A detailed description of the in situ polymerization of polymerizable mesogenic compounds can be found in D.J.Broer et al., Makromolekulare Chemie 190, 2255 (1989).

The inventive polymerizable liquid crystalline mixtures comprise preferably 0.001 to 15 %, in particular 0.01 to 8 % and very particularly preferably 0.1 to 5 % by weight of non-polymerizable chiral compounds of formula Ic.

Polymerizable liquid crystalline mixtures are preferred that comprise 1 to 3 chiral compounds of formula Ic.

If polymerizable chiral compounds of formula I are present in the inventive polymerizable liquid crystalline mixtures, these compounds can also be used in the inventive polymerizable liquid crystalline mixtures in higher amounts than given above for the non-polymerizable chiral compounds of formula Ic.

In a preferred embodiment of the present invention the polymerizable liquid crystalline mixtures are comprising 1 to 80 % by weight, preferably 2 to 60 %, in particular 5 to 40 % by weight of a polymerizable chiral compound of formula Ic comprising at least one polymerizable functional group.

Of the polymerizable liquid crystalline mixtures comprising one or polymerizable chiral compounds of formula Ic with one polymerizable functional groups (= monofunctional compounds), particularly preferred are those comprising 1 to 60 %, in particular 2 to 45 %, very preferably 3 to 35 % by weight of a polymerizable chiral monofunctional compound of formula Ic.

Of the polymerizable liquid crystalline mixtures comprising one or polymerizable chiral compounds of formula Ic with two polymerizable functional groups ( = bifunctional compounds), particularly preferred are those comprising 1 to 50 %, in particular 2 to 35 %, very preferably 3 to 25 % by weight of a polymerizable chiral bifunctional compound of formula Ic.

The inventive polymerizable liquid crystalline mixtures can additionally comprise one or more other suitable components, such as, for example, catalysts, sensitizers, stabilizers, co-reacting monomers or surface-active compounds.

In a preferred embodiment of the invention, the inventive polymerizable liquid crystalline mixture comprises a stabilizer that is used to prevent undesired spontaneous polymerization for example during storage of the composition. As stabilizers in principal all compounds can be used that are known to the skilled in the art for this purpose. These compounds are commercially available in a broad variety. Typical examples for stabilizers are 4-ethoxyphenol or butylated hydroxytoluene (BHT).

It is also possible, in order to increase crosslinking of the polymers, to add up to 20% of a non mesogenic compound with two or more polymerizable functional groups to the polymerizable composition alternatively or additionally to the multifunctional polymerizable mesogenic compounds.

Typical examples for difunctional non mesogenic monomers are alkyldiacrylates or alkyldimethacrylates with alkyl groups of 1 to 20 C atoms. Typical examples for non mesogenic monomers with more than two polymerizable groups are trimethylpropanetrimethacrylate or pentaerythritoltetraacrylate.

Polymerization of inventive compositions comprising compounds with only one polymerizable functional group leads to linear polymers, whereas in the presence of compounds with more than one polymerizable functional group crosslinked polymers are obtained.

For the preparation of anisotropic polymer gels, the liquid crystalline mixtures can be polymerized in situ as described above, however, in this case alignment of the polymerizable mixture is not necessary.

The inventive chiral compounds of formula Ic can also be used for the prepration of thermochromic liquid crystalline mixtures. Such mixtures are characterized in that they exhibit a chiral liquid crystalline phase or chiral mesophase, like e.g. a chiral smectic phase or a chiral nematic (= cholesteric) phase, with a helically twisted molecular structure that shows selective reflection of a specific waveband of light, wherein the pitch of the molecular helix and thereby the reflected wavelengths are depending on the temperature.

Especially preferred are inventive liquid crystalline mixtures with thermochromic behaviour that exhibit a cholesteric phase. Of these preferred compositions, further preferred are compositions that exhibit a cholesteric phase and a smectic phase, most preferably a chiral smectic phase, at temperatures below the temperature range of the cholesteric phase. The inventive liquid crystalline mixtures exhibiting thermochromic behaviour can be polymerizable or non-polymerizable.

From the foregoing description, one skilled in the art can easily ascertain the essential characteristics of this invention, and without departing from the spirit and scope thereof, can make various changes and modifications of the invention to adapt it to various usages and conditions.

Without further elaboration, it is believed that one skilled in the art can, using the preceding description, utilize the present invention to ist fullest extent. The following examples are, therefore, to be construed as merely illustrative

and not limitative of the remainder of the disclosure in any way whatsoever.

In the foregoing and in the following examples, unless otherwise indicated, all temperatures are set forth uncorrected in degrees Celsius and all parts and percentages are by weight

The values of the helical twisting power HTP of a chiral compound in a liquid crystalline host are given according to the equation HTP = $(p \cdot c)^{-1}$ in $\mu m^{-1}$, wherein p is the pitch of the molecular helix, given in µm, and c is the concentration by weight of the chiral compound in the host given in relative values (thus, e.g. a concentration of 1 % by weight is corresponding to a value of c of 0.01).

The following abbreviations are used to illustrate the liquid crystalline phase behaviour of the compounds: K = crystalline; N = nematic; S = smectic; Ch = cholesteric; I = isotropic. The numbers between these symbols indicate the phase transition temperatures in degree Celsius.

In addition, the following abbreviations are used

DCC = dicyclohexylcarbodiimide
DCM = dichloromethane
HTP = helical twisting power
mp = melting point

Example 1

Compound (1 ) was prepared according to reaction scheme 1

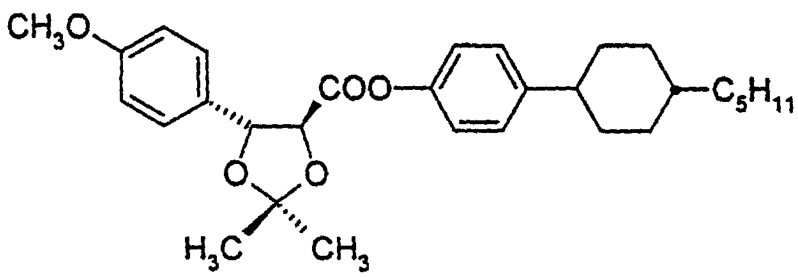

(1)

(E)-3-(4-methoxyphenyl)-2-propenoate (1.1)

Trans-Methoxycinnamic acid (17.8 g, 100 mmol), sulphuric acid (2 ml) and methanol (200 ml) were stirred under reflux for 2 hours. The mixture was cooled, concentrated in vacuo, white crystals precipitated and these were isolated by vacuum filtration to give methyl (E)-3-(4-methoxyphenyl)-2-propenoate (16.1 g, 84 %). $^1$H-NMR gave the expected signals.

3R,2S-Methyl 2,3-dihydroxy-3-(4-methoxyphenyl)propanoate (1.2)

$[DHQD]_2$PHAL (54.5 mg, 0.25 mol %), methyl (E)-3-(4-methoxyphenyl)-2-propenoate (5.38 g, 28 mmol), N-methyl morpholine N-oxide (60% aqueous solution, 7.0 ml) and tert-butanol (11.2 ml) were charged to a 100 ml 3-neck flask and stirred at room temperature. Potassium osmate (VI) dihydrate (21.4 mg, 0.2 mol %) was added portionwise and the reaction was stirred at room temperature. The reaction procedure was monitored by HPLC. After 17 hours, Tiron (200 mg) was added to quench the reaction, followed by water (50 ml). The mixture was allowed to stir for one hour, ethyl acetate (50 ml) was added to the mixture and the resultant two layers were vigorously stirred for 30 minutes. The organic phase was removed and washed with water, dried ($Na_2SO_4$) and evaporated to dryness on a rotary evaporator. The product was purified by flash column chromatography using DCM: ethyl acetate (3:2) as eluant. Evaporation of the appropriate fractions gave methyl 2,3-dihydroxy-3-(4-methoxyphenyl)propanoate as a white crystalline solid (3.2 g, 62%). mp. 108°C, $[\alpha]_D$ = -1.2° (THF, conc. = 0.01306, 22°C). $^1$H- NMR was in accordance with expected signals.

Methyl 5-(4-methoxyphenyl)-2,2-dimethyl-1,3-dioxolane 4-carboxylate (1.3)

Methyl 2,3-dihydroxy-3-(4-methoxyphenyl)propanoate (3.5 g, 15.5 mmol), copper sulphate anhydrous (4.3 g, 27 mmol, 1.8 equiv.) and acetone (40 ml, excess) were stirred in the presence of a catalytic amount of para-toluene sulphonic acid at 35°C. After 16 hours, the copper sulphate was removed by filtration, and the filtrate was removed

and evaporated to dryness on a rotary evaporator. The product was purified by flash column chromatography using DCM as eluant. Evaporation of the appropriate fractions left methyl 5-(4-methoxyphenyl)-2,2-dimethyl -1,3-dioxolane 4-carboxylate as a clear, colourless oil (4.0 g, 96 %). $[\alpha]_D$ = +37.4° (THF, conc. = 0.01712, 20°C). $^1$H-NMR was in accordance with expected signals.

(5R,4S)-5-(4-methoxyphenyl)2,2-dimethyl-1,3-dioxolane-4-carboxylic acid (1.4)

Methyl 5-(4-methoxyphenyl)-2,2-dimethyl-1,3-dioxolane 4-carboxylate (2.0 g, 7.5 mmol) and aqueous sodium hydroxide solution (0.36 g in 20 ml) and industrial methylated spirits (20 ml) were stirred at 80°C for 3 hours. The mixture was cooled to room temperature, neutralised with dilute hydrochloric acid and concentrated in vacuo. The product was extracted with DCM, dried over $NaSO_4$ and evaporated to dryness to leave (5R,4S)-5-(4-methoxyphenyl) 2,2-dimethyl-1,3-dioxolane-4-carboxylic acid as a colourless oil (1.7 g, 90 %). $^1$H-NMR was in accordance with expected signals. I.R. showed expected peaks. ,

(5R,4S)-5-(4-methoxyphenyl)2,2-dimethyl-1,3-dioxolane-4-carbonyloxycyclohexyl-4-phenyl-4'-pentane (1)

(5R,4S)-5-(4-Methoxyphenyl)2,2-dimethyl-1,3-dioxolane-4-carboxylic acid (1.6 g, 6.3 mmol), DCC (1.44 g, 7.0 mmol), 4-(4'-pentylcyclohexyl)phenol (1.5) (1.55g. 6.3 mmol) and a catalytic amount of dimethylamino pyridine were stirred at room temperature in dichloromethane (40 ml). After 3 hours, the precipitate of dicyclohexyl urea was removed by filtration, the filtrate was washed with water, dried ($Na_2SO_4$) and evaporated to dryness. The product was purified by flash column chromatography using DCM as eluant to leave (5R,4S)-5-(4-methoxyphenyl)2,2-dimethyl-1,3-dioxolane-4-carbonyloxycyclohexyl-4-phenyl-4'-pentane (1), a clear, colourless oil on evaporation of the appropriate fractions. Yield = 1.6 g, 53 %. mp. 56°C. $[\alpha]_D$ = +89.9° (THF, conc. = 0.01124, 25°C). $^1$H-NMR was in accordance with expected signals.

Example 2

Compound (2) was prepared according to reaction scheme 1.

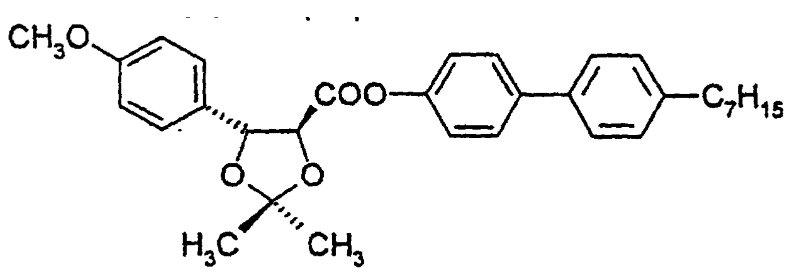

(2)

(5R,4S)-5-(4-Methoxyphenyl)2,2-dimethyl-1,3-dioxolane-4-carboxylic acid (1.4) (0.8 g, 3.2 mmol), DCC (0.7 g, 3.5 mmol), 4-(4'-heptylphenyl)phenol (1.6) (0.8 g, 3.2 mmol) and a catalytic amount of dimethylamino pyridine were stirred at room temperature in dichloromethane (40 ml). After 3 hours, the precipitate of dicyclohexyl urea was removed by filtration, the filtrate was washed with water, dried ($Na_2SO_4$) and evaporated to dryness. The product was purified by flash column chromatography using DCM as eluant to leave (5R,4S)-5-(4-methoxyphenyl)2,2-dimethyl-1,3-dioxolane-4-carbonyloxyphenyl-4-phenylheptane (2) as a clear, colourless oil on evaporation of the appropriate fractions. Yield = 0.8 g, 50 %. Mp. 60°C. $[\alpha]_D$ = +103.7° (THF, conc. = 0.01326, 25°C). $^1$H NMR was in accordance with expected signals.

Example 3

Compound (3) was prepared according to reaction scheme 1.

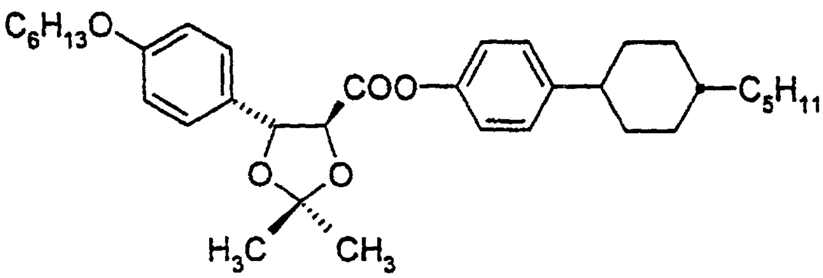 (3)

The trans methyl 4-alkoxycinnamate (homologue of compound 1.1) in the first step was prepared by Williamson etherification of trans methyl 4-hydroxycinnamate with the corresponding bromoalkane.

Methyl (E)-3-(4-hexyloxyphenyl)-2-propenoate (3.1)

Trans methyl 4-hydroxycinnamate (20.0 g, 11.2 mmol), 1-bromohexane (18.5 g, 112 mmol) and potassium carbonate (16.6 g, 120 mmol) were stirred under reflux in butanone. The mixture was allowed to cool after 16 hours, evaporated to dryness and the residue was partitioned between DCM and water. The DCM layer was removed and dried ($Na_2SO_4$) and evaporated to leave a yellow solid methyl (E)-3-(4-hexyloxyphenyl)-2-propenoate which was recrystallized from petroleum. Yield = 20.0 g, 71 %.

Methyl 2,3-dihydroxy-3-(4-hexyloxyphenyl)propanoate (3.2)

Synthesis and purification were carried out as described in example 1. Methyl 2,3-dihydroxy-3-(4-hexyloxyphenyl)propanoate was obtained as a colourless oil which crystallized on standing (3.4 g, 62%). $^1$H-NMR was in accordance with expected signals.

(5R,4S)-methyl 5-(4-hexyloxyphenyl)-2,2-dimethyl-1,3-dioxolane 4-carboxylate (3.3)

Synthesis and purification were carried out as described in example 1. (5R,4S)-methyl 5-(4-methoxyphenyl)-2,2-dimethyl-1,3-dioxolane 4-carboxylate was obtained as an oil (2.5 g, 69 %). $[\alpha]_D$ = +36.5° (THF, conc. = 0.01768, 19°C). $^1$H-NMR was in accordance with expected signals. The HTP was 2 $\mu m^{-1}$, measured in the commercially available nematic host mixture BL 087 (from Merck Ltd., Poole, UK) at a concentration of 6.34 % by weight.

(5R,4S)-5-(4-hexyloxyphenyl)2,2-dimethyl-1,3-dioxolane-4-carboxylic acid (3.4)

Synthesis and purification were carried out as described in example 1. (5R,4S)-5-(4-hexyloxyphenyl)2,2-dimethyl-1,3-dioxolane-4-carboxylic acid was obtained as a yellow oil (1.7 g, 90 %). $^1$H-NMR and I.R. spectra were in accordance with expected signals. $[\alpha]_D$ = +22.5° (THF, conc. = 0.01731, 19°C).

(5R,4S)-5-(4-hexyloxyphenyl)2,2-dimethyl-1,3-dioxolane-4-carbonyloxycyclohexyl-4-phenyl-4'-pentane (3)

Synthesis and purification were carried out as described in example 1. (5R,4S)-5-(4-methoxyphenyl)2,2-dimethyl-1,3-dioxolane-4-carbonyloxycyclohexyl-4-phenyl-4'-pentane (3) was obtained as a clear oil. Yield = 59 %. $[\alpha]_D$ = +112.8° (THF, conc. = 0.01037, 22°C). $^1$H-NMR was in accordance with expected signals.

The HTP of (3) was 14.9 $\mu m^{-1}$, measured in the commercially available nematic host mixture BL 087 (from Merck Ltd., Poole, UK) at a concentration of 5.11 % by weight.

The preceding examples can be repeated with similar success by substituting the generically or specifically described reactants and/or operating conditions of this invention for those used in the preceding examples.

## Claims

1. Chiral compounds of formula Ic

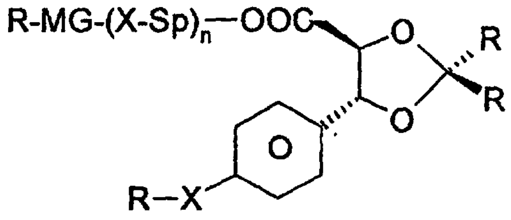

Ic

wherein

X is in each case independently -O-, -S-, -CO-, -COO-, -OCO-, -OCO-O-, -CO-NH-, -NH-CO-, $-OCH_2-$, $-CH_2O-$, -CH=CH-, -C≡C-, $-SCH_2-$, $-CH_2S-$, -CH=CH-COO-, -OOC-CH=CH- or a single bond,

Sp is in each case independently a spacer group with 1 to 20 C atoms,

n is 0 or 1,

MG is a mesogenic group, and

R is in each case independently H, CN, halogen or a straight-chain or branched alkyl radical with up to 25 C atoms which may be unsubstituted, mono- or polysubstituted by halogen or CN, it being also possible for one or more non-adjacent $CH_2$ groups to be replaced, in each case independently from one another, by -O-, -S-, -NH-, $-N(CH_3)-$, -CO-, -COO-, -OCO-,-OCO-O-, -S-CO-, -CO-S- or -C≡C- in such a manner that oxygen atoms are not linked directly to one another, or denoting $P-(Sp-X)_n-$, with Sp, X and n having one of the meanings given above and P being a polymerizable group.

**2.** Chiral compounds according to claim 1, wherein MG is selected of formula II

$$-(A^1-Z)_m-A^2- \qquad \text{II}$$

wherein

Z is -O-, -S-, -CO-, -COO-, -OCO-, -CO-NH-, -NH-CO-, $-CH_2CH_2-$, $-OCH_2-$, $-CH_2O-$, $-SCH_2-$, $-CH_2S-$, -CH=CH-, -CH=CH-COO-, -OCO-CH=CH-, -C≡C- or a single bond,

$A^1$ and $A^2$ are in each case independently selected from

a) 1,4-phenylene in which, in addition, one or more CH groups may be replaced by N,

b) 1,4-cyclohexylene in which, in addition, one or two non-adjacent $CH_2$ groups may be replaced by O and/or S,

c) 1,3-dioxolane-4,5-diyl,

d) 1,4-cyclohexenylene, 1,4-bicyclo-(2,2,2)-octylene, piperidine-1,4-diyl, naphthalene-2,6-diyl, decahydronaphthalene-2,6-diyl, or 1,2,3,4-tetrahydronaphthalene-2,6-diyl,

it being possible for all these groups to be unsubstituted, mono- or polysubstituted with halogen, cyano or nitro groups or alkyl, alkoxy, alkylcarbonyl or alkoxycarbonyl groups with 1 to 7 C atoms, wherein one or more H atoms may be substituted by F or Cl, and

m is 0, 1, 2 or 3.

**3.** Chiral compounds according to claim 1 or 2, wherein MG is selected of the following formulae

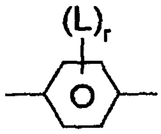

IIa

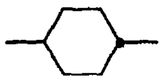

IIb

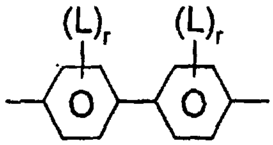

IIc

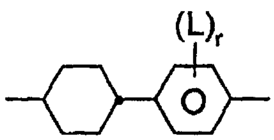

IId

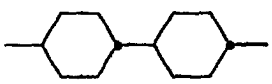

IIe

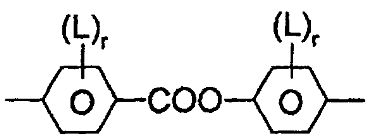

IIf

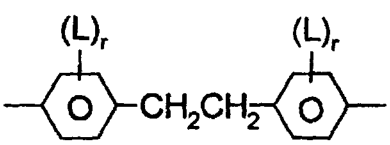

IIg

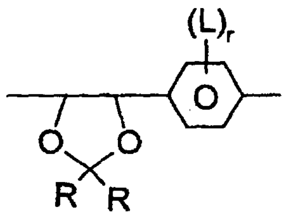

IIh

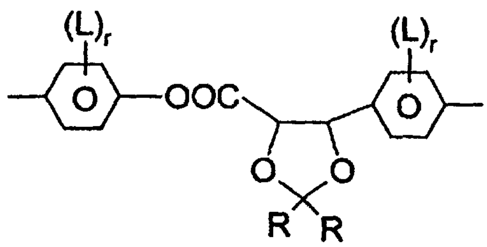

IIi

wherein R has the meaning of formula Ic, L is F, Cl, CN or an optionally fluorinated alkyl, alkoxy or alkanoyl group with 1 to 4 C atoms, and r is 0, 1 or 2.

**4.** Chiral compounds according to at least one of claims 1 to 3, wherein R is alkyl or alkoxy with 1 to 12 C atoms.

**5.** Chiral compounds according to at least one of claims 1 to 4, wherein at least one of the groups R is denoting $P-(Sp-X)_n-$, with P, Sp, X and n having each independently one of the meanings given in formula Ic.

**6.** A liquid crystalline mixture comprising at least one chiral compound according to at least one of claims 1 to 5.

**7.** A liquid crystalline mixture according to claim 6, further comprising at least one polymerizable mesogenic compound having at least one polymerizable functional group.

**8.** A chiral linear or crosslinked liquid crystalline polymer obtainable by polymerizing a mixture according to claim 7.

**9.** Use of a chiral compound, mixture or polymer according to at least one of claims 1 to 8 in liquid crystal displays, STN, TN, AMD-TN, temperature compensation, guest-host, phase change or surface stabilized or polymer stabilized cholesteric texture (SSCT, PSCT) displays, in active and passive optical elements, polarizers, compensators, alignment layers, colour filters or holographic elements, in adhesives, synthetic resins with anisotropic mechanical properties, cosmetics, diagnostics, liquid crystal pigments, for decorative and security applications, in nonlinear optics, optical information storage or as chiral dopants.

**10.** A liquid crystal display comprising a liquid crystalline mixture according to claim 6 or 7.

**11.** A liquid crystal display according to claim 10, **characterized in that** it is an SSCT or PSCT display.

**12.** A polarizer, compensator, alignment layer, colour filter or liquid crystal pigment comprising a chiral compound, mixture or polymer according to at least one of claims 1 to 8.

**Patentansprüche**

**1.** Chirale Verbindungen der Formel Ic

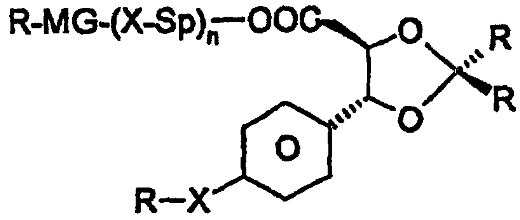

Ic

worin

X jeweils unabhängig -O-, -S-, -CO-, -COO-, -OCO-, -OCO-O-,-CO-NH-. -NH-CO-, $-OCH_2-$, $-CH_2O-$, -CH=CH-, -C≡C-, $-SCH_2-$ , $-CH_2S-$, -CH=CH-COO-, -OOC-CH=CH- oder eine Einfachbindung bedeutet,

Sp jeweils unabhängig eine Spacergruppe mit 1 bis 20 C-Atomen bedeutet,

n 0 oder 1 bedeutet,

MG eine mesogene Gruppe bedeutet und

R jeweils unabhängig H, CN, Halogen oder einen geradkettigen oder verzweigten Alkylrest mit bis zu 25 C-Atomen bedeutet, der unsubstituiert oder ein- oder mehrfach mit Halogen oder CN substituiert sein kann, wobei auch eine oder mehrere nicht benachbarte $CH_2$-Gruppen jeweils unabhängig voneinander durch -O-, -S-, -NH-, $-N(CH_3)-$, -CO-, -COO-, -OCO-, -OCO-O-, -S-CO-, -CO-S- oder -C≡C- so ersetzt sein können, dass Sauerstoffatome nicht direkt miteinander verknüpft sind, oder auch $P-(Sp-X)_n-$ bedeutet, wobei Sp, X und n eine der obengenannten Bedeutungen haben und P eine polymerisierbare Gruppe ist.

**2.** Chirale Verbindungen nach Anspruch 1, worin MG ausgewählt ist aus der Formel II

$$-(A^1-Z)_m-A^2- \qquad \text{II}$$

worin

Z -O-, -S-, -CO-, -COO-, -OCO-, -CO-NH-, -NH-CO-, $-CH_2CH_2-$, - $OCH_2-$, $-CH_2O-$, $-SCH_2-$. $-CH_2S-$, -CH=CH-, -CH=CH-COO-, -OCO-CH=CH-, -C≡C- oder eine Einfachbindung bedeutet,

$A^1$ und $A^2$ jeweils unabhängig ausgewählt sind aus

a) 1,4-Phenylen, in dem eine oder mehrere CH-Gruppen auch durch N ersetzt sein können,

b) 1,4-Cyclohexylen, in dem eine oder zwei nicht benachbarte $CH_2$-Gruppen auch durch O oder S ersetzt sein können,

c) 1,3-Dioxolan-4,5-diyl,

d) 1,4-Cyclohexenylen, 1,4-Bicyclo(2,2,2)octylen, Piperidin-1,4-diyl, Naphthalin-2,6-diyl, Decahydronaphthalin-2,6-diyl oder 1,2,3,4-Tetrahydronaphthalin-2,6-diyl,

wobei alle diese Gruppen jeweils unsubstituiert oder ein- oder mehrfach mit Halogen-, Cyan- oder Nitrogruppen oder Alkyl-, Alkoxy-, Alkylcarbonyl- oder Alkoxycarbonylgruppen mit 1 bis 7 C-Atomen substituiert sein können, worin ein oder mehrere H-Atome durch F oder Cl substituiert sein können, und

m 0, 1, 2 oder 3 bedeutet.

**3.** Chirale Verbindungen nach Anspruch 1 oder 2, worin MG aus den folgenden Formeln ausgewählt ist

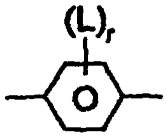

IIa

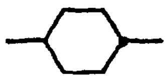

IIb

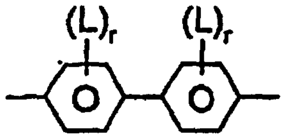

IIc

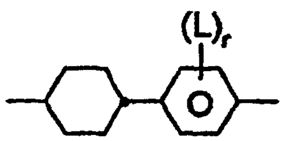

IId

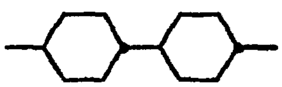

IIe

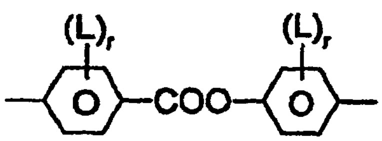

IIf

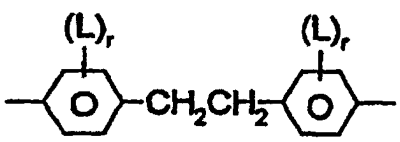

IIg

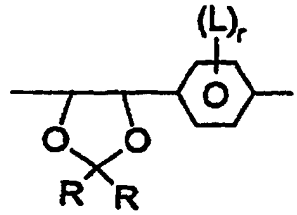

IIh

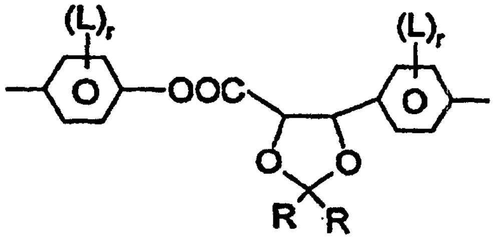

IIi

worin R die bei der Formel Ic angegebene Bedeutung hat, L F, Cl, CN oder eine gegebenenfalls fluorierte Alkyl-, Alkoxy- oder Alkanoylgruppe mit 1 bis 4 C-Atomen bedeutet und r 0, 1 oder 2 bedeutet.

**4.** Chirale Verbindungen nach mindestens einem der Ansprüche 1 bis 3, worin R Alkyl oder Alkoxy mit 1 bis 12 C-Atomen ist.

**5.** Chirale Verbindungen nach mindestens einem der Ansprüche 1 bis 4, worin mindestens eine der Gruppen R P-(Sp-X)$_n$- bedeutet, wobei P, Sp, X und n jeweils unabhängig eine der in Formel Ic angegebenen Bedeutungen haben.

**6.** Flüssigkristalline Mischung enthaltend mindestens eine chirale Verbindung nach mindestens einem der Ansprüche 1 bis 5.

**7.** Flüssigkristalline Mischung nach Anspruch 6, weiterhin enthaltend mindestens eine polymerisierbare mesogene Verbindung mit mindestens einer polymerisierbaren funktionellen Gruppe.

**8.** Chirales lineares oder vernetztes flüssigkristallines Polymer erhältlich durch Polymerisieren einer Mischung nach Anspruch 7.

**9.** Verwendung einer chiralen Verbindung, chiralen Mischung oder eines chiralen Polymers nach mindestens einem der Ansprüche 1 bis 8 in Flüssigkristallanzeigen, STN-, TN-, AMD-TN-, Temperaturkompensations-, Guest-Host-, Phasenwechselanzeigen oder Anzeigen mit oberflächen- oder polymerstabilisierter cholesterischer Textur (SSCT, PSCT), in aktiven und passiven optischen Elementen, Polarisatoren, Kompensatoren, Orientierungsschichten, Farbfiltern oder holographischen Elementen, in Klebstoffen, Kunstharzen mit anisotropen mechanischen Eigenschaften, Kosmetika, Diagnostika, Flüssigkristallpigmenten, für dekorative und Sicherheitsanwendungen, in der nichtlinearen Optik, in der optischen Datenspeicherung oder als chirale Dotierstoffe.

**10.** Flüssigkristallanzeige enthaltend eine flüssigkristalline Mischung nach Anspruch 6 oder 7.

**11.** Flüssigkristallanzeige nach Anspruch 10, **dadurch gekennzeichnet, dass** es sich um eine SSCT- oder PSCT-Anzeige handelt.

**12.** Polarisator, Kompensator, Orientierungsschicht, Farbfilter oder Flüssigkristallpigment enthaltend eine chirale Verbindung, chirale Mischung oder ein chirales Polymer nach mindestens einem der Ansprüche 1 bis 8.

**Revendications**

**1.** Composés chiraux de formule Ic :

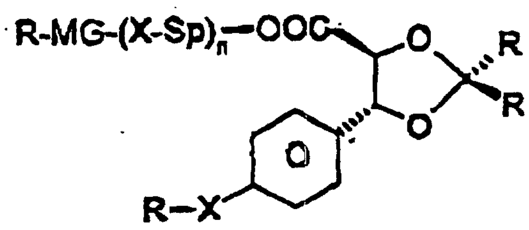

Ic

dans laquelle

X représente indépendamment dans chaque cas -O-, -S-, -CO-, -COO-, -OCO-, -OCO-O-, -CO-NH-, -NH-CO-, $-OCH_2-$, $-CH_2O-$, -CH=CH-, -C≡C-, $-SCH_2-$, $-CH_2S-$, -CH-CH-COO-,-OOC-CH=CH- ou une simple liaison,

Sp représente indépendamment dans chaque cas un groupement espaceur avec 1 à 20 atomes de carbone,

n vaut 0 ou 1,

MG est un groupement mesogénique, et

R représente indépendamment dans chaque cas H, CN, un halogène ou un radical alkyle à chaîne linéaire ou ramifiée avec jusqu'à 25 atomes de carbone qui peuvent ne pas être substitués, mono- ou poly-substitués par un halogène ou CN, il est aussi possible pour un ou plusieurs groupements $CH_2$ non adjacents d'être remplacés, indépendamment dans chaque cas, par -O-, -S-, -NH-,$-N(CH_3)-$, -CO-, -COO-, -OCO-, -OCO-O-, -S-CO-, -CO-S- ou -C≡C- de telle manière que les atomes d'oxygène ne soient pas liés directement les uns aux autres, ou désignant $P-(Sp-X)_n-$ avec Sp, X et n ayant l'une des significations données précédemment et P étant un groupement polymérisable.

**2.** Composés chiraux selon la revendication 1, dans lesquels MG est choisi selon la formule II:

$$-(A^1-Z)_m-A^2- \qquad \text{II}$$

dans laquelle

Z représente-O-, -S-, -CO-, -COO-, -OCO-, -CO-NH-, -NH-CO-, $-CH_2CH_2-$, $-OCH_2-$, $-CH_2O-$, $-SCH_2-$, $-CH_2S-$, -CH=CH-,-CH=CH-COO-, -OCO-CH=CH- , -C≡C- ou une simple liaison,

$A^1$ et $A^2$ sont chacun indépendamment choisis parmi :

a) le 1,4-phénylène dans lequel, en outre, un ou plusieurs groupements CH peuvent être remplacés par N,
b) le 1,4-cyclohexylène dans lequel, en outre, un ou plusieurs groupements $CH_2$ non adjacents peuvent être remplacés par O et/ou S,
c) le 1,3-dioxolan-4,5-diyle,
d) le 1,4-cyclohexenylène, le 1,4-bicyclo-(2,2,2)-octylène, le pipéridin-l,4-diyle, le naphtalèn-2,6-diyle, le décahydronaphtalén-2,6-diyle ou le 1,2,3,4-tétrahydronaphtalèn-2,6-diyle,
il est possible pour tous ces groupements de ne pas être substitués, d'être mono- ou poly-substitués avec un halogène, des groupements cyano ou nitro ou des groupements alkyle, alcoxy, alkylcarbonyle ou alcoxycarbonyle avec de 1 à 7 atomes de carbone, dans lesquels un ou plusieurs atomes d'hydrogène peuvent être substitués par F ou Cl, et
m vaut 0, 1, 2 ou 3.

**3.** Composés chiraux selon les revendications 1 ou 2, dans lesquels MG est choisi parmi les formules suivantes :

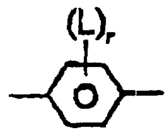

IIa

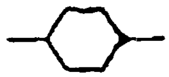

IIb

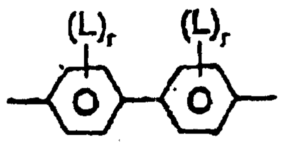

IIc

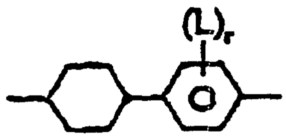

IId

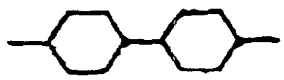

IIe

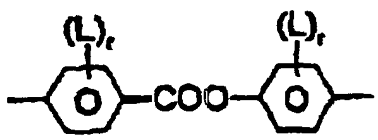

IIf

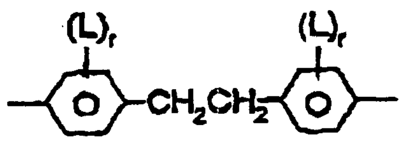

IIg

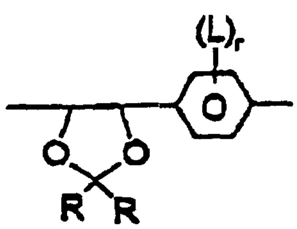

IIh

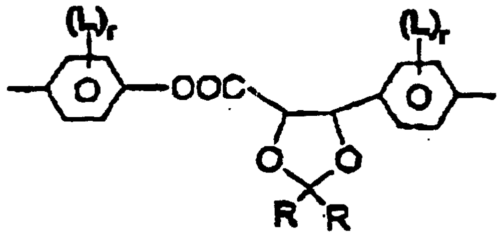

IIi

dans lesquels R a la signification de la formule Ic, L représente F, Cl CN ou un groupement alkyle, alcoxy ou alcanoyle facultativement fluorés, avec 1 à 4 atomes de carbone, et r vaut 0, 1 ou 2.

**4.** Composés chiraux selon l'une quelconque des revendications 1 à 3, dans lesquels R est un groupement alkyle, ou un alcoxy avec 1 à 12 atomes de carbone.

**5.** Composés chiraux selon l'une quelconque des revendications 1 à 4, dans lesquels au moins l'un des groupements R désigne P-(Sp-X)$_n$-, avec P, Sp, X et n ayant chacun indépendamment l'une des significations données pour la formule Ic.

**6.** Mélange cristallin liquide comprenant au moins un composé chiral selon l'une quelconque des revendications 1 à 5.

**7.** Mélange cristallin liquide selon la revendication 6, comprenant, en outre, au moins un composé mesogénique polymérisable ayant au moins un groupement fonctionnel polymérisable.

**8.** Polymère cristallin liquide chiral, linéaire ou réticulé susceptible d'être obtenu en polymérisant un mélange selon la revendication 7.

**9.** Utilisation d'un composé, d'un mélange ou d'un polymère chiral, selon l'une quelconque des revendications 1 à 8 dans des écrans cristallins liquides, STN, TN, AMD-TN, la compensation de température, l'hôte, les écrans à texture cholestérique à changement de phase ou stabilisé par la surface ou stabilisé par un polymère (SSCT, PSCT), dans des éléments optiques actifs et passifs, des polarisateurs, des compensateurs, des couches d'alignement, des filtres colorés ou des éléments holographiques, dans des adhésifs, des résines synthétiques avec des propriétés mécaniques anisotropes, des cosmétiques, des éléments de diagnostique, des pigments cristallins liquides, pour des applications décoratives et de sécurité, dans des optiques non linéaires, du stockage d'information optique ou des dopants chiraux.

**10.** Écran cristallin liquide comprenant un mélange cristallin liquide selon les revendications 6 ou 7.

**11.** Écran cristallin liquide selon la revendication 10, **caractérisé en ce qu'**il s'agit d'un écran SSCT ou PSCT.

**12.** Polarisateur, compensateur, couche d'alignement, filtre coloré ou pigment cristallin liquide comprenant un composé, un mélange ou un polymère chiral selon l'une quelconque des revendications 1 à 8.